# EUROPÄISCHE PATENTSCHRIFT

(11) **EP 2 684 719 B1**
(45) Veröffentlichungstag und Bekanntmachung des Hinweises auf die Patenterteilung: **27.12.2017**
(21) Anmeldenummer: 13175933.4
(22) Anmeldetag: 10.07.2013
(51) Int. Cl.: B60H 3/00, A61L 9/12

(54) **Beduftungsvorrichtung**
Fragrance device
Dispositif de diffusion de fragrance

(30) Priorität: 13.07.2012 DE 102012212369
(43) Veröffentlichungstag der Anmeldung: 15.01.2014
(73) Patentinhaber: MAHLE Behr GmbH & Co. KG, 70469 Stuttgart (DE)
(72) Erfinder: Baumann, Tobias, 71720 Oberstenfeld (DE); Lochmahr, Dipl.-Ing. (FH) Karl, 71665 Vaihingen/Enz (DE); Pitz, Dipl.-Ing. (FH) Eric, 70199 Stuttgart (DE); Rais, Dr. rer. nat. Thomas, 71672 Marbach/Neckar (DE)
(74) Vertreter: Grauel, Andreas

(56) Entgegenhaltungen:
- EP-A1- 2 143 577
- EP-A1- 2 145 788
- EP-A2- 2 085 258

## Beschreibung

### Technisches Gebiet

Die Erfindung betrifft eine Beduftungsvorrichtung, insbesondere zum Einsatz in einer Klimaanlage für Kraftfahrzeuge, mit einer Aufnahmevorrichtung, einer Duftmittelkartusche, einem Aktuator und einer Klappe, wobei die Duftmittelkartusche in die Aufnahmevorrichtung einsetzbar ist und eine erste Öffnung aufweist, die durch die Klappe in zumindest einer Stellung der Klappe verschließbar ist. Weiterhin betrifft die Erfindung eine diesbezügliche Anordnung.

### Stand der Technik

Um den höheren Anforderung der Kunden an Komfort gerecht zu werden, weisen moderne Klimaanlagen, insbesondere für den Einsatz in Kraftfahrzeugen, zunehmend Einrichtungen auf, die es erlauben, die in den Fahrgastraum geleitete Luft zu beduften.

Im Stand der Technik sind Systeme bekannt, bei denen eine Kartusche, welche zumindest einen Duftstoff enthält, mittels einer Klappe verschlossen werden und die Klappe durch den Einsatz von Schrittmotoren geöffnet und geschlossen werden kann. Hierzu ist der Schrittmotor über eine Kinematik mit der Klappe, welche die Kartusche verschließt, verbunden. Über den Grad der Öffnung der Klappe lässt sich hierbei die Intensität der Beduftung der Luft steuern.

Die DE 103 28 747 A1 offenbart eine Vorrichtung zur Beduftung eines Luftstroms in einer Klimaanlage.

Die EP 2 143 577 A1 offenbart eine Beduftungsvorrichtung, die mit einer reversiblen Verschlusseinrichtung ausgebildet ist. Durch eine Mechanik kann beim Einschieben der Duftmittelvorratsvorrichtung in den Aufnahmeraum die Verschlussklappe automatisch geöffnet werden.

Nachteilig an der Vorrichtung gemäß dem Stand der Technik ist insbesondere der Einsatz von Schrittmotoren, welche das Gewicht und den Bauraumbedarf einer Beduftungsvorrichtung erhöhen. Außerdem erhöht die notwendige Kinematik das Systemgewicht und den Bauraumbedarf weiter. Eventuell notwendige Getriebe zur Umwandlung der Bewegung des Schrittmotors können außerdem akustische Störgeräusche erzeugen.

### Darstellung der Erfindung, Aufgabe, Lösung, Vorteile

Daher ist es die Aufgabe der vorliegenden Erfindung eine Beduftungsvorrichtung und eine diesbezügliche Anordnung bereitzustellen, welche ohne den Einsatz einer gesonderten Kinematik die Beduftung eines Luftstroms erlaubt. Außerdem soll die Beduftungsvorrichtung einen im Vergleich zum Stand der Technik geringeren Bauraumbedarf und ein geringeres Systemgewicht aufweisen, sowie akustisch vorteilhafte Eigenschaften aufweisen.

Die Aufgabe der vorliegenden Erfindung wird hinsichtlich der Beduftungsvorrichtung durch eine Beduftungsvorrichtung mit den Merkmalen gemäß Anspruch 1 gelöst.

Ein Ausführungsbeispiel der Erfindung betrifft eine Beduftungsvorrichtung, insbesondere zum Einsatz in einer Klimaanlage für Kraftfahrzeuge, mit einer Aufnahmevorrichtung, einer Duftmittelkartusche, einem Aktuator und einer Klappe, wobei die Duftmittelkartusche in die Aufnahmevorrichtung einsetzbar ist und eine erste Öffnung aufweist, die durch die Klappe in zumindest einer Stellung der Klappe verschließbar ist und eine erste Öffnung aufweist, die durch die Klappe in zumindest einer Stellung der Klappe verschließbar ist, wobei jede Klappe durch jeweils einen Aktuator betätigbar ist zum Öffnen und Schließen der Klappe, wobei der Aktuator einteilig mit der Duftmittelkartusche ausgebildet ist.

Einteilig bedeutet, dass der Aktuator mit der Duftmittelkartusche fest und entweder wieder lösbar oder nicht lösbar verbunden ist, etwa durch Verclipsen, Verschrauben, Verkleben, Vercrimpen, Verschweißen, Verklemmen oder Vernieten.

Durch ein solches Beduftungssystem kann ein Duftmittel in den Luftstrom einer Klimaanlage eingebracht werden, und es kann damit die zur Klimatisierung verwendete Luft beduftet werden. Durch ansteuerbare Aktuatoren kann die Menge des freigesetzten Duftmittels gezielt beeinflusst werden und eine situationsgerechte Beduftung erzeugt werden.

Auch ist es vorteilhaft, wenn der Aktuator durch einen Piezoaktuator oder ein Memory-Metall-Element gebildet ist. Dies erlaubt eine einfache und kostengünstige Gestaltung.

Piezoaktuatoren sowie Memory-Metall-Elemente zeichnen sich durch einen äußerst geringen Bauraumbedarf und ein niedriges Gewicht aus. Außerdem lassen sich beide Arten von Aktuatoren unkompliziert ansteuern.

Weiterhin ist es zu bevorzugen, wenn die Duftmittelkartusche oder der Aktuator eine Steuereinheit aufweist, über welche der Aktuator ansteuerbar ist.

Über eine Steuereinheit an der Duftmittelkartusche bzw. am Aktuator selbst lässt sich der Aktuator besonders einfach ansteuern, da kein zusätzliches dezentrales Steuergerät benötigt wird. Im Falle einer Steuereinheit direkt am Aktuator ist auch insbesondere der Austausch eines Aktuators einfach zu bewerkstelligen.

Auch ist es zweckmäßig, wenn der Aktuator eine lineare und/oder rotatorische Bewegung der Klappe verursacht.

Über entweder eine lineare, eine rotatorische oder eine kombinierte Bewegung lässt sich das Öffnen und Schließen der Duftmittelkartusche besonders vorteilhaft darstellen. So kann eine Duftmittelkartusche beispielsweise über eine Klappe, welche als Schieber ausgebildet ist, durch das translatorische Verschieben der Klappe oder das rotatorische verdrehen der Klappe geöffnet und verschlossen werden. Weiterhin kann auch eine Klappe, welche an einem Umlenkpunkt drehbar gelagert ist, von einem Aktuator so bewegt werden, derart dass eine Öffnung in der Duftmittelkartusche freigegeben oder verschlossen wird, etwa wenn die Klappe um den Umlenkpunkt verdreht wird.

Auch ist es zweckmäßig, wenn die freigegebene Querschnittsfläche der Öffnung direkt von dem Arbeitsweg des Aktuators abhängig ist.

Dies ist insbesondere der Fall, wenn der Aktuator nicht über zwischengeschaltete Übersetzungen an die Klappe angebunden ist und so der Arbeitsweg direkt auch den Öffnungsweg der Klappe bestimmt, was zu einer besonders einfachen Konstruktion beiträgt.

Gemäß einem weiteren vorteilhaften Ausführungsbeispiel, ist es zu bevorzugen, wenn die Duftmittelkartusche mit der Klappe und dem Aktuator eine verbaubare einteilig ausgeführte Einheit bildet.

Hierdurch ist die Möglichkeit gegeben, die Baueinheit, also die Duftmittelkartusche, den Aktuator und die Klappe, in einem auszutauschen, beispielsweise im Falle eines Defektes oder einer leeren Duftmittelkartusche. Die Zuordnung des Aktuators und der Klappe zu einer Duftmittelkartusche als Einheit bringt den Vorteil mit sich, dass bei einer vorhandenen Mehrzahl von Beduftungsvorrichtungen jede Beduftungsvorrichtung für sich auswechselbar ist, ohne dabei in einer Wechselwirkung mit den anderen Beduftungsvorrichtungen zu stehen.

Auch ist es zweckmäßig, wenn der Aktuator so angeordnet ist, dass er eine Krafteinwirkung zwischen der Duftmittelkartusche und der Klappe oder der Aufnahmevorrichtung und der Klappe hervorruft.

Durch die Krafteinwirkung des Aktuators direkt auf die Duftmittelkartusche und die Klappe bzw. die Aufnahmevorrichtung und die Klappe wird die Klappe direkt durch den Aktuator bewegt, ohne dass zusätzliche Getriebe oder Gestänge als Kinematik zwischengeschaltet sind. Dadurch wird die Konstruktion weniger defektanfällig und reduziert zusätzlich die benötigten Bauteile, den benötigten Bauraum sowie das Gesamtgewicht.

Dies ist insbesondere dann von Vorteil, wenn die Einbaulage der Beduftungsvorrichtung keine Möglichkeit bietet, dass der Aktuator direkt auf die Duftmittelkartusche und die Klappe einwirkt. Ebenfalls vorteilhaft ist dies, wenn die Klappe und der Aktuator nicht mit der Duftmittelkartusche als eine verbaubare Einheit ausgeführt sind.

Der Aktuator und die Klappe könnten dann beispielsweise in der Klimaanlage, einem Luftkanal oder insbesondere in einem eigenständigen Beduftungssystem montierbar sein, so dass nur die Duftmittelkartusche als austauschbares Teil in eine Aufnahmevorrichtung eingesetzt wird. Je nach Ausführung des Aktuators und den zur Verfügung stehenden Platzverhältnissen kann sich hierfür ein Vorteil hinsichtlich der Austauschkosten ergeben.

Auch ist es zu bevorzugen, wenn der Aktuator durch einen Draht gebildet ist, welcher aus einer Form-Gedächtnis-Legierung erzeugt ist.

Eine Form-Gedächtnis-Legierung oder ein sogenanntes Memory-Metall sind insbesondere vorteilhaft, da sie auf einfache Weise ansteuerbar sind und einen geringen Bauraumbedarf aufweisen. Form-Gedächtnis-Legierungen weisen darüber hinaus ein sehr hohes spezifisches Arbeitsvermögen auf. Durch die Veränderung der Temperatur der Form-Gedächtnis-Legierung kann ein Element in einen vorbestimmten Zustand versetzt werden. Durch ein Abkühlen kann entsprechend wieder der Ausgangszustand erreicht werden. Neben der hohen Leistungsfähigkeit zeichnen sich Form-Gedächtnis-Legierungen auch durch eine hohe Anzahl an möglichen Arbeitszyklen aus. Aktuatoren aus Form-Gedächtnis-Legierungen sind daher besonders dauerhaltbar.

Darüber hinaus ist es vorteilhaft, wenn der Draht mit einer wieder lösbaren Verbindung mit der Kartusche und/oder der Aufnahmevorrichtung und/oder der Klappe verbunden ist.

Eine wieder lösbare Verbindung ist insbesondere vorteilhaft, um eine möglichst hohe Reparatur- und Wartungsfreundlichkeit zu erreichen. So kann über eine wieder lösbare Verbindung beispielsweise erreicht werden, dass auch nur einzelne Elemente ausgetauscht werden können. Beispielsweise kann vorgesehen werden, dass bei einem Austausch der Kartusche der Aktuator in oder an der Beduftungsvorrichtung verbleibt.

Die Aufgabe der Anordnung wird gelöst mit den Merkmalen von Anspruch 10.

Ein Ausführungsbeispiel betrifft dabei eine Anordnung einer Beduftungsvorrichtung oder mehrerer Beduftungsvorrichtungen, in einer Klimaanlage oder einem Luftkanal oder einem Beduftungssystem, wobei der Aktuator eine Krafteinwirkung zwischen der Klappe und der Aufnahmevorrichtung oder zwischen der Klappe und einem Bauteil der Klimaanlage oder des Luftkanals oder des Beduftungssystems hervorruft.

Dies ist insbesondere dann vorteilhaft, wenn eine Mehrzahl von Duftmittelkartuschen in einer Klimaanlage verbaut ist. Ein Austausch einzelner Duftmittelkartuschen ist dann kostengünstig zu realisieren, da nur die Duftmittelkartuschen ausgetauscht werden müssen, die tatsächlich leer sind.

Darüber hinaus ist es zweckmäßig, wenn eine Anordnung von einer Mehrzahl von Duftmittelkartuschen vorgesehen ist, wobei die Duftmittelkartuschen einzeln oder gemeinsam austauschbar sind.

Dies ist insbesondere vorteilhaft, da dadurch der Austausch einzelner Duftmittelkartuschen kostengünstig zu realisieren ist, da nur die Duftmittelkartuschen ausgetauscht werden müssen, die tatsächlich leer sind.

Außerdem ist es vorteilhaft, wenn die Aktuatoren der Duftmittelkartuschen individuell oder im Verbund ansteuerbar sind.

Durch die Ansteuerbarkeit der Duftmittelkartuschen einzeln oder im Verbund kann eine besonders individuelle Regelung der Duftmittelabgabe erreicht werden. Weiterhin vorstellbar ist eine Verwendung unterschiedlicher Duftmittel in unterschiedlichen Duftmittelkartuschen, die dann nach externer Vorgabe wohldosiert zusammen in den Luftstrom der Klimaanlage freigesetzt werden.

Vorteilhafte Weiterbildungen der vorliegenden Erfindung sind in den Unteransprüchen beschrieben.

### Kurze Beschreibung der Zeichnungen

Im Folgenden wird die Erfindung anhand eines Ausführungsbeispiels unter Bezugnahme auf die Zeichnung detailliert erläutert. In der Zeichnung zeigen:
- Fig.1: eine Darstellung einer Beduftungsvorrichtung im Schnitt in einer Seitenansicht,
- Fig. 2: eine weitere Darstellung einer alternativen Ausführung einer Beduftungsvorrichtung mit geschlossener Klappe,
- Fig. 3: eine Darstellung der Beduftungsvorrichtung aus Figur 2 mit geöffneter Klappe,
- Fig. 4: eine weitere Ausführungsform einer Beduftungsvorrichtung mit geschlossener Klappe, und
- Fig. 5: eine Darstellung der Beduftungsvorrichtung aus Figur 4 mit geöffneter Klappe.

### Bevorzugte Ausführung der Erfindung

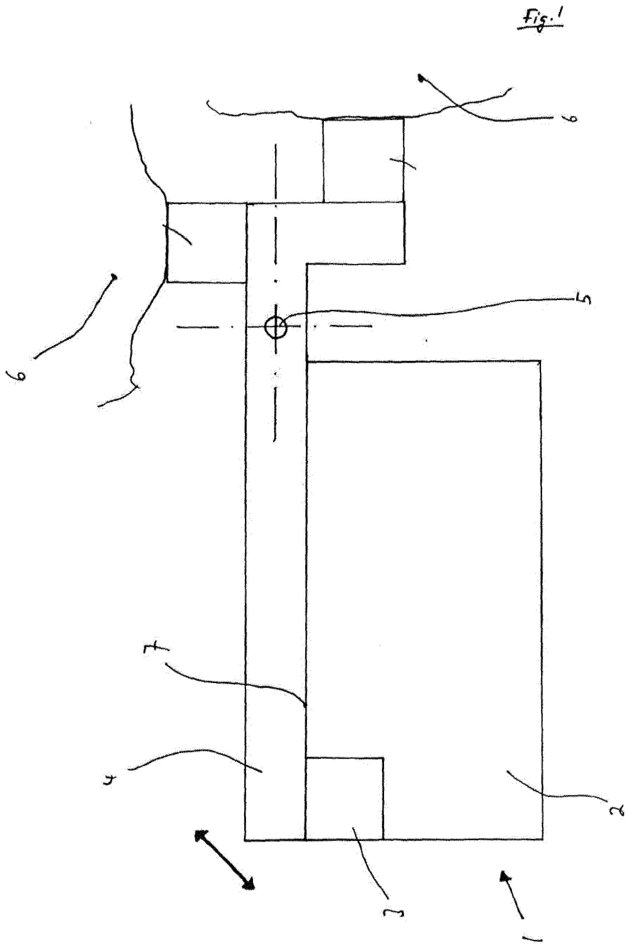

Die Figur 1 zeigt eine seitliche Ansicht einer Beduftungsvorrichtung 1. Die Beduftungsvorrichtung 1 besteht im Wesentlichen aus einer Duftmittelkartusche 2, einem Aktuator 3 sowie einer Klappe 4.

Die Duftmittelkartusche 2 ist mit einem Duftmittel gefüllt, welches durch ein Verstellen der Klappe 4 aus der Duftmittelkartusche 2 dosiert austreten kann. Zur Betätigung der Klappe 4 ist in der Beduftungsvorrichtung 1 ein Aktuator 3 vorgesehen.

In der erfindungsgemäßen Ausführungsform, entsprechend der Figur 1, wirkt der Aktuator 3 direkt auf die Klappe 4. Direkt heißt in diesem Falle, dass zwischen dem Aktuator 3 und der Klappe 4 keine weiteren Umlenkmechanismen, wie etwa Getriebe oder andere Hebemechanismen als Kinematik vorgesehen sind. Dies reduziert den notwendigen Bauraum der Beduftungsvorrichtung 1 und senkt deren Gesamtgewicht.

Der Aktuator 3 ist im Ausführungsbeispiel der Erfindung durch ein Memory-Metall-Element oder durch einen Piezoaktuator gebildet.

In der Figur 1 sind für den Aktuator 3 drei alternative Einbaupositionen dargestellt. In der ersten Einbauposition ist der Aktuator 3 auf der Seitenwand der Duftmittelkartusche 2 angebracht. Der Aktuator 3 stützt sich zum einen auf der Seitenwand der Duftmittelkartusche 2 und zum anderen an der Klappe 4 ab. Somit erzeugt der Aktuator 3 eine Krafteinwirkung auf die Klappe 4, die sich dadurch relativ zur Duftmittelkartusche 2 bewegen kann.

Die Klappe 4 ist in der Figur 1 als eine L-förmige Klappe ausgeführt, welche an einem Umlenkpunkt 5 drehbar gelagert ist und einen langen und einen kurzen Schenkel aufweist.

Der Aktuator 3 ist einteilig mit der Duftmittelkartusche 2 ausgeführt.

Einteilig ausgeführt bedeutet dabei, dass der Aktuator 3 mit der Duftmittelkartusche 2 fest verbunden ist. Diese Verbindung ist entweder lösbar oder alternativ nicht lösbar.

Die Verbindung des Aktuators 3 mit der Duftmittelkartusche 2 kann beispielsweise durch Verschrauben, Verclipsen, Verkleben oder Vernieten erfolgen.

In der Einbauposition des Aktuators 3, die in der Figur 1 dargestellt ist, wird die Klappe 4 durch die Krafteinwirkung des Aktuators 3 um den Umlenkpunkt 5 verdreht, wodurch die Öffnung 7 am oberen Rand der Duftmittelkartusche 2 freigegeben wird.

Über den Arbeitsweg des Aktuators 3 kann der Öffnungswinkel der Klappe 4 vorgegeben werden. Dadurch ist eine gute Dosierung des Duftmittels, welches durch die Öffnung 7 der Duftmittelkartusche 2 austritt, möglich. Die Positionierung des Aktuators 3 unmittelbar an der Duftmittelkartusche 2 begünstigt es, das Beduftungssystem 1 als eine gemeinsam verbaubare Einheit auszuführen, welche in ihrer Gesamtheit ausgetauscht werden kann. In diesem Ausführungsbeispiel besteht die austauschbare Einheit im Wesentlichen aus der Duftmittelkartusche 2, dem direkt an der Duftmittelkartusche 2 positionierten Aktuator 3 und der Klappe 4.

Grundsätzlich wird das Beduftungssystem 1 in eine Aufnahmevorrichtung, welche im Inneren der Klimaanlage angeordnet ist, eingesteckt und derart in der Klimaanlage oder in einem Luftkanal positioniert.

Auch ist es denkbar, dass nur die Duftmittelkartusche 2 und der Aktuator 3 als Einheit austauschbar sind. Die Klappe 4 würde in diesem Falle fest installiert in der Klimaanlage oder dem Luftkanal verbleiben.

Es ist darüber hinaus auch denkbar, mehrere Beduftungssysteme 1, wie sie in der Figur 1 dargestellt sind, parallel in einer Klimaanlage oder einem Luftkanal anzuordnen. Vorteilhaft ist dabei, dass jede Duftmittelkartusche 2 mittels eines eigenen Aktuators 3 betätigt werden kann und die Komponenten je nach Positionierung innerhalb der Klimaanlage oder des Luftkanals einzeln austauschbar sind.

In alternativen Ausführungsformen einer Beduftungsvorrichtung 1 sind auch von der hier gezeigten Geometrie der Klappe 4 abweichende Gestaltungen der Klappe 4 vorsehbar. So kann eine Klappe nicht um einen Umlenkpunkt verdrehbar gestaltet sein, sondern sie kann durch eine lineare Bewegung eines Aktuators, ähnlich einem Schieber, über der Duftmittelkartusche verschoben werden, um die Öffnung an der Duftmittelkartusche freizugeben oder zu verschließen. Ebenso kann eine Klappe vorgesehen werden, die durch einen rotatorisch arbeitenden Aktuator von der Öffnung der Duftmittelkartusche weggedreht wird.

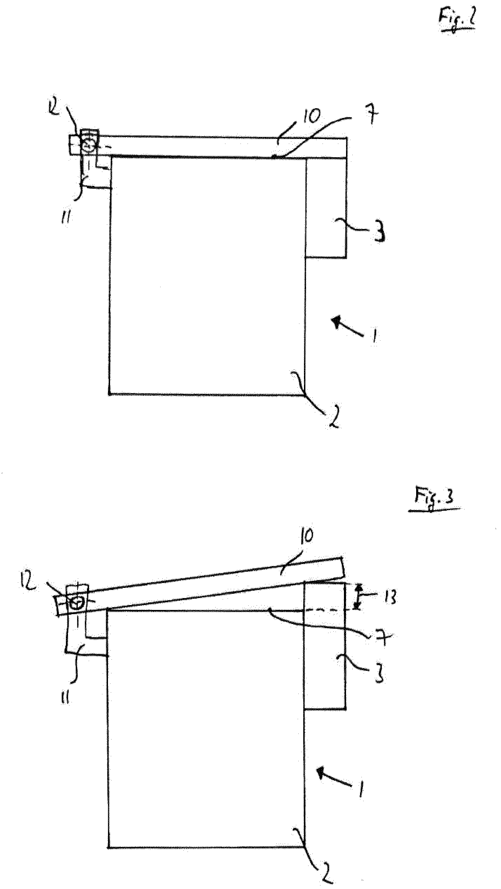

Die Figur 2 zeigt ein weiteres alternatives Ausführungsbeispiel einer Beduftungsvorrichtung 1, dieses besteht auch im Wesentlichen aus der Duftmittelkartusche 2 und einem Aktuator 3. Der Aktuator 3 ist an einer Außenfläche der Duftmittelkartusche 2 positioniert. Die Klappe 10, welche die oben an der Duftmittelkartusche angeordnete Öffnung 7 der Duftmittelkartusche 2 verschließt, ist über einen Umlenkpunkt 12, in welchem die Klappe mit einem Halter 11 verbunden ist, drehbar gelagert. Der Halter 11 ist in dem gezeigten Ausführungsbeispiel an einer Außenfläche der Duftmittelkartusche 2 befestigt.

Der Aktuator 3 erzeugt eine translatorische, nach oben, in einer Richtung senkrecht zur Ebene der Öffnung, gerichtete Bewegung, wodurch die Klappe 10 um den Umlenkpunkt 12 verdreht wird. Dadurch wird die Klappe verkippt und öffnet die Öffnung 7.

Die Figur 3 zeigt die Beduftungsvorrichtung 1 der Figur 2, wobei in Figur 3 die Duftmittelkartusche geöffnet ist.

Der translatorische Arbeitsweg des Aktuator 3 ist in Figur 3 mit dem Bezugszeichen 13 dargestellt. Die Vorderkante der Klappe 10 wird durch das Betätigen des Aktuators 3 nach oben geschoben und dabei wird die Klappe 10 um den Umlenkpunkt 12 verdreht. Auf diese Weise entsteht, ähnlich dem in Figur 1 gezeigten Prinzip, eine Schrägstellung der Klappe 10, wodurch die am oberen Ende der Duftmittelkartusche 2 angeordnete Öffnung 7 freigegeben wird.

Der Arbeitsweg 13 des Aktuators 3 hat direkten Einfluss auf den Öffnungswinkel der Klappe 10 und damit auch auf die freigegebene Fläche der Öffnung 7 der Duftmittelkartusche 2. Das heißt, ein geringer Arbeitsweg 13 führt zu einer geringen Öffnung 7 der Duftmittelkartusche 2, ein großer Arbeitsweg 13 führt entsprechend zu einer großen Öffnung 7.

Die in den Figuren 2 und 3 gezeigte Beduftungsvorrichtung 1 ist als eine verbaubare Einheit ausgeführt und kann in einem Stück ausgetauscht werden.

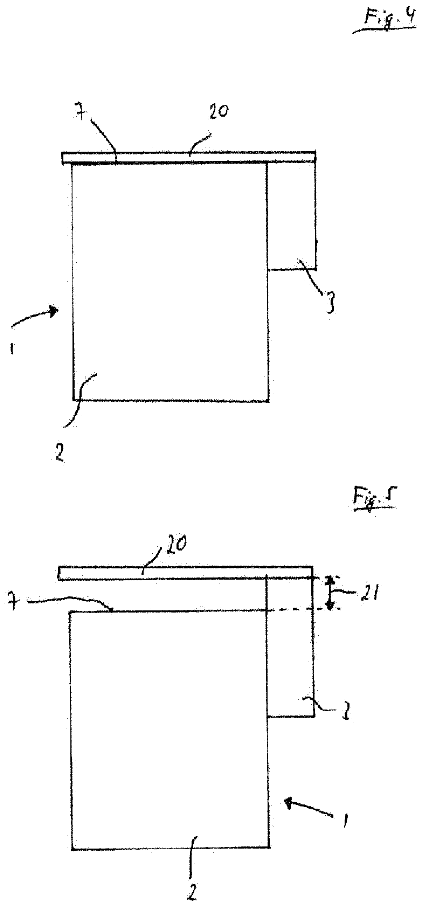

Die Figur 4 und die Figur 5 zeigen eine weitere alternative Ausführungsform einer Beduftungsvorrichtung 1. Dabei zeigt die Figur 4 den geschlossenen Zustand und die Figur 5 den geöffneten Zustand der Beduftungsvorrichtung 1.

In der Beduftungsvorrichtung 1 der Figur 4 ist der Aktuator 3 wieder an einer der Außenflächen der Duftmittelkartusche 2 positioniert. Der Aktuator 3 steht in direktem Kontakt mit der Klappe 20, welche die im oberen Bereich der Duftmittelkartusche 2 angeordnete Öffnung 7 verschließt. Durch eine Betätigung des Aktuators 3 wird die Klappe 20, wie es in Figur 5 dargestellt ist, nach oben verfahren, wodurch die Öffnung 7, welche im oberen Bereich der Duftmittelkartusche 2 angeordnet ist, freigegeben wird.

Der Arbeitsweg des Aktuators 3 ist in Figur 5 mit dem Bezugszeichen 21 gekennzeichnet. Der Grad der Öffnung 7 der Duftmittelkartusche 2 hängt direkt mit dem Arbeitsweg 21 des Aktuators 3 zusammen. Je größer der Arbeitsweg des Aktuators 3 ist, umso weiter wird die Klappe 20 von der Duftmittelkartusche 2 abgehoben.

In alternativen Ausführungsformen für das Beduftungssystem 1 sind, wie in Figur 1 schon angedeutet, auch weitere Ausführungen der Klappe bzw. des Aktuators denkbar. Neben den hier gezeigten translatorischen Bewegungen ist auch eine rotatorische Bewegung, zum Beispiel durch den Einsatz von Memory-Metall-Elementen oder von Piezoaktuatoren vorsehbar.

Eine Steuereinheit zur Ansteuerung des Aktuators 3 ist in den oben beschriebenen Ausführungsformen entweder direkt in den Aktuator 3 integriert oder an die Duftmittelkartusche 2 angebunden oder auf sonstige Weise in oder an der Beduftungsvorrichtung angeordnet.

Es ist in alternativen Ausführungsformen auch vorteilhaft, die Steuereinheit für den Aktuator an der Aufnahmevorrichtung, in welche die Duftmittelkartusche innerhalb der Klimaanlage oder eines Luftkanals eingesteckt wird, anzuordnen.

Um den Aktuator ansteuern zu können, ist eine elektrische Kontaktierung des Aktuators vorzusehen. Dies kann beispielsweise über einen Stecker oder eine andere Schnittstelle erfolgen. Die Steuereinheit kann entsprechend auch auf der in der Klimaanlage oder in dem Luftkanal verbleibenden Seite der Kontaktierung angeordnet sein.

Es ist vorsehbar, dass über ein zentrales Steuergerät bzw. eine zentrale Steuereinheit eine Mehrzahl von Aktuatoren von Kartuschen angesteuert werden kann. Die Ansteuerbarkeit eines Aktuators bedeutet insbesondere, dass dieser steuerbar und/oder regelbar ist.

## Patentansprüche

1. Beduftungsvorrichtung (1) mit einer Aufnahmevorrichtung, einer Duftmittelkartusche (2), einem Aktuator (3) und einer Klappe (4, 10, 20), wobei die Duftmittelkartusche (2) in die Aufnahmevorrichtung einsetzbar ist und eine erste Öffnung (7) aufweist, die durch die Klappe (4, 10, 20) in zumindest einer Stellung der Klappe (4, 10, 20) verschließbar ist, die Klappe (4, 10, 20) durch den Aktuator (3) zum Öffnen und Schließen der Klappe (4, 10, 20) betätigbar ist, **dadurch gekennzeichnet, dass** der Aktuator (3) einteilig mit der Duftmittelkartusche (2) ausgebildet ist.

2. Beduftungsvorrichtung (1) nach Anspruch 1, **dadurch gekennzeichnet, dass** der Aktuator (3) durch einen Piezoaktuator oder ein Memory-Metall-Element gebildet ist.

3. Beduftungsvorrichtung (1) nach Anspruch 1 oder 2, **dadurch gekennzeichnet, dass** die Duftmittelkartusche (2) oder der Aktuator (3) eine Steuereinheit aufweist, über welche der Aktuator (3) ansteuerbar ist.

4. Beduftungsvorrichtung (1) nach einem der vorhergehenden Ansprüche 1 bis 3, **dadurch gekennzeichnet, dass** der Aktuator (3) eine lineare und/oder rotatorische Bewegung der Klappe (4, 10, 20) verursacht.

5. Beduftungsvorrichtung (1) nach einem der vorhergehenden Ansprüche 1 bis 4, **dadurch gekennzeichnet, dass** die freigegebene Querschnittsfläche der Öffnung (7) direkt von dem Arbeitsweg des Aktuators (3) abhängig ist.

6. Beduftungsvorrichtung (1) nach einem der vorhergehenden Ansprüche, **dadurch gekennzeichnet, dass** die Duftmittelkartusche (2) mit der Klappe (4, 10, 20) und dem Aktuator (3) eine verbaubare einteilig ausgeführte Einheit bildet.

7. Beduftungsvorrichtung (1) nach einem der vorhergehenden Ansprüche, **dadurch gekennzeichnet, dass** der Aktuator (3) so angeordnet ist, dass er eine Krafteinwirkung zwischen der Duftmittelkartusche (2) und der Klappe (4, 10, 20) oder der Aufnahmevorrichtung und der Klappe (4, 10, 20) hervorruft.

8. Beduftungsvorrichtung nach einem der vorhergehenden Ansprüche, **dadurch gekennzeichnet, dass** der Aktuator durch einen Draht gebildet ist, welcher aus einer Form-Gedächtnis-Legierung erzeugt ist.

9. Beduftungsvorrichtung nach Anspruch 8, **dadurch gekennzeichnet, dass** der Draht mit einer wieder lösbaren Verbindung mit der Kartusche und/oder der Aufnahmevorrichtung und/oder der Klappe verbunden ist.

10. Anordnung einer Beduftungsvorrichtung oder mehrerer Beduftungsvorrichtungen (1) nach einem der vorhergehenden Ansprüche 1 bis 9 in einer Klimaanlage oder einem Luftkanal oder einem Beduftungssystem, **dadurch gekennzeichnet, dass** der Aktuator (3) eine Krafteinwirkung zwischen der Klappe (4, 10, 20) und der Aufnahmevorrichtung oder zwischen der Klappe (4, 10, 20) und einem Bauteil der Klimaanlage oder des Luftkanals oder des Beduftungssystems hervorruft.

11. Anordnung nach Anspruch 10, **dadurch gekennzeichnet, dass** eine Mehrzahl von Duftmittelkartuschen vorgesehen sind, wobei die Duftmittelkartuschen (2) einzeln oder gemeinsam austauschbar sind.

12. Anordnung nach einem der vorhergehenden Ansprüche 10 oder 11, **dadurch gekennzeichnet, dass** die Aktuatoren (3) der Duftmittelkartuschen (2) individuell oder im Verbund ansteuerbar sind.

## Claims

1. A fragrancing device (1) with an acceptance element, a fragrance cartridge (2), an actuator (3) and a flap (4, 10, 20), wherein the fragrance cartridge (2) can be inserted into the acceptance element and has a first opening (7) which can be closed by the flap (4, 10, 20) in at least one position of the flap (4, 10, 20), wherein the flap (4, 10, 20) is operable by the actuator (3) in order to open and close the flap (4, 10, 20), **characterised in that** the actuator (3) is integrally formed with the fragrance cartridge (2).

2. The fragrancing device (1) according to claim 1, **characterised in that** the actuator (3) is formed by a piezo actuator or a memory metal element.

3. The fragrancing device (1) according to claim 1 or 2, **characterised in that** the fragrance cartridge (2) or the actuator (3) has a control unit via which the actuator (3) can be controlled.

4. The fragrancing device (1) according to one of the preceding claims 1 to 3, **characterised in that** the actuator (3) causes a linear and/or rotatory movement of the flap (4, 10, 20).

5. The fragrancing device (1) according to one of the preceding claims 1 to 4, **characterised in that** the unblocked cross-sectional area of the opening (7) is directly dependent from the working path of the actuator (3).

6. The fragrancing device (1) according to one of the preceding claims, **characterised in that** the fragrance cartridge (2) together with the flap (4, 10, 20) and the actuator (3) forms an installable unit of a single-piece design.

7. The fragrancing device (1) according to one of the preceding claims, **characterised in that** the actuator (3) is arranged such that it causes an application of a force between the fragrance cartridge (2) and the flap (4, 10, 20) or the acceptance element and the flap (4, 10, 20).

8. The fragrancing device according to one of the preceding claims, **characterised in that** the actuator is formed by a wire which is created from a shape memory alloy.

9. The fragrancing device according to claim 8, **characterised in that** the wire is connected to the cartridge and/or the acceptance element and/or the flap via a detachable connection.

10. An arrangement of a fragrancing device or several fragrancing devices (1) according to one of the preceding claims 1 to 9 in an air-conditioning system or an air channel or a fragrancing system, **characterised in that** the actuator (3) causes an application of force between the flap (4, 10, 20) and the acceptance element or between the flap (4, 10, 20) and a component of the air-conditioning system or the air channel or the fragrancing system.

11. The arrangement according to claim 10, **characterised in that** a plurality of fragrance cartridges is provided, wherein the fragrance cartridges (2) can be exchanged individually or together.

12. The arrangement according to one of the preceding claims 10 or 11, **characterised in that** the actuators (3) of the fragrance cartridges (2) can be controlled individually or in combination.

## Revendications

1. Dispositif servant à parfumer (1) comprenant un dispositif récepteur, une cartouche de substance parfumée (2), un actionneur (3) et un volet (4, 10, 20), où la cartouche de substance parfumée (2) peut être introduite dans le dispositif récepteur et présente une première ouverture (7) qui, dans au moins une position du volet (4, 10, 20), peut être fermée par le volet (4, 10, 20), le volet (4, 10, 20), pour l'ouverture et la fermeture du volet (4, 10, 20), pouvant être actionné par l'actionneur (3), **caractérisé en ce que** l'actionneur (3) est conçu en formant une seule et même pièce avec la cartouche de substance parfumée (2).

2. Dispositif servant à parfumer (1) selon la revendication 1, **caractérisé en ce que** l'actionneur (3) est formé par un actionneur piézoélectrique ou par un élément métallique à mémoire de forme.

3. Dispositif servant à parfumer (1) selon la revendication 1 ou 2, **caractérisé en ce que** la cartouche de substance parfumée (2) ou l'actionneur (3) présente une unité de commande par laquelle peut être commandé l'actionneur (3).

4. Dispositif servant à parfumer (1) selon l'une quelconque des revendications précédentes 1 à 3, **caractérisé en ce que** l'actionneur (3) provoque un mouvement linéaire et / ou de rotation du volet (4, 10, 20).

5. Dispositif servant à parfumer (1) selon l'une quelconque des revendications précédentes 1 à 4, **caractérisé en ce que** la surface de section dégagée de l'ouverture (7) est directement dépendante de la course de fonctionnement de l'actionneur (3).

6. Dispositif servant à parfumer (1) selon l'une quelconque des revendications précédentes, **caractérisé en ce que** la cartouche de substance parfumée (2) forme, avec le volet (4, 10, 20) et avec l'actionneur (3), un ensemble compatible réalisé d'une seul tenant.

7. Dispositif servant à parfumer (1) selon l'une quelconque des revendications précédentes, **caractérisé en ce que** l'actionneur (3) est disposé de manière telle, qu'il déclenche un effet de force se produisant entre la cartouche de substance parfumée (2) et le volet (4, 10, 20), ou bien entre le dispositif récepteur et le volet (4, 10, 20).

8. Dispositif servant à parfumer selon l'une quelconque des revendications précédentes, **caractérisé en ce que** l'actionneur est formé par un fil qui est produit à partir d'un alliage à mémoire de forme.

9. Dispositif servant à parfumer selon la revendication 8, **caractérisé en ce que** le fil est relié à la cartouche et / ou au dispositif récepteur et / ou au volet par une liaison à nouveau détachable.

10. Agencement d'un dispositif servant à parfumer ou de plusieurs dispositifs servant à parfumer (1) selon l'une quelconque des revendications précédentes 1 à 9, dans un système de climatisation ou dans un conduit d'air, ou bien dans un système servant à parfumer, **caractérisé en ce que** l'actionneur (3) déclenche un effet de force se produisant entre le volet (4, 10, 20) et le dispositif récepteur, ou bien entre le volet (4, 10, 20) et un composant du système de climatisation ou du conduit d'air ou du système servant à parfumer.

11. Agencement selon la revendication 10, **caractérisé en ce qu'**il est prévu une pluralité de cartouches de substance parfumée, où les cartouches de substance parfumée (2) peuvent être remplacées individuellement ou collectivement.

12. Agencement selon l'une des revendications précédentes 10 ou 11, **caractérisé en ce que** les actionneurs (3) des cartouches de substance parfumée (2) peuvent être commandés individuellement ou de façon combinée.
